# EUROPEAN PATENT SPECIFICATION

(11) **EP 2 234 548 B1**
(45) Date of publication and mention of the grant of the patent: **04.05.2016**
(21) Application number: 08866763.9
(22) Date of filing: 19.12.2008
(51) Int. Cl.: A61F 2/01

(54) **IMPROVED DEVICES AND METHODS FOR EMBOLUS REMOVAL DURING ACUTE ISCHEMIC STROKE**
VERBESSERTE VORRICHTUNGEN UND VERFAHREN FÜR EMBOLUSENTFERNUNG WÄHREND EINES AKUTEN ISCHÄMISCHEN SCHLAGANFALLS
DISPOSITIFS ET PROCÉDÉS AMÉLIORÉS POUR LE RETRAIT D'UN EMBOLE AU COURS D'UN ACCIDENT VASCULAIRE CÉRÉBRAL ISCHÉMIQUE AIGU

(30) Priority: 19.12.2007 US 15154 P; 11.04.2008 US 44392 P; 19.05.2008 US 123390; 30.05.2008 US 57613 P; 30.07.2008 US 182370
(43) Date of publication of application: 06.10.2010
(73) Proprietor: Covidien LP, Mansfield, MA 02048 (US)
(72) Inventor: FERRERA, David, A., Redondo Beach CA 90278 (US); FULKERSON, John, Rancho Santa Margarita CA 92688 (US)
(74) Representative: Kehoe, Laura Ellen
(86) International application number: PCT/US2008/087759
(87) International publication number: WO 2009/086154

(56) References cited:
- WO-A2-2007/089897
- US-A1- 2002 095 141
- US-A1- 2004 153 117
- US-A1- 2005 055 047
- US-A1- 2005 125 023
- US-A1- 2005 126 979
- US-A1- 2006 122 643
- US-A1- 2007 288 054

## Description

### BACKGROUND OF THE INVENTION

The present invention relates to minimally invasive and catheter delivered embolic capture devices for use in the vasculature, especially those suited for usage in the brain and vessel systems perfusing the same.

Several acute stroke therapy trials were reported in 2007. Interventional Management of Stroke (IMS) II found a 9.9% rate of symptomatic intracerebral hemorrhage after combination IV-intra-arterial (IA) tissue-type plasminogen activator therapy was administered within 3 hours of acute stroke onset.

Further results from the MERCI and Multi-MERCl investigators have shown that successful recanalization of acute distal ICA and proximal middle cerebral artery occlusions can he achieved in 53% to 63% of patients using the MERC1 retriever (Concentric Medical) alone or in combination with 1 V/IA thrombolytics, yet symptomatic hemorrhages occurred in 6% of those patients who recanalized.

Another approach in those who fail MERCI retrieval has been to use angioplasty or self-expanding stents to compress friable clot and allow better penetration of thrombolytic agents. Recanalization rates of up to 89% with balloons and 79% with stents have been achieved in small numbers of patients.

Reports of aggressive revascularization of acute ICA and tandem ICA/middle cerebral artery occlusions using stents and intracranial thrombolysis have demonstrated the feasibility of this approach, again in small numbers of patients.

Attempts to identify prognostic factors for hemorrhagic complications and eventual clinical outcomes in patients undergoing these aggressive multimodal interventions showed that residual distal occlusions, tandem occlusions, larger initial pretreatment CT infarct size by Alberta Stroke Program Early CT Score (ASPECTS) score, hyperglycemia and use of both IA and IV thromholytics were all associated with negative results. Novel mechanical revascularization strategies used deflated microballoon catheters and the Alligator retrieval device (Chestnut Medical Technologies) to open vessels that may be refractory to IA thrombolytics or the MERCI device.

There are new stents being used to assist in the coiling of wide-necked aneurysms, such as the closed-cell design Enterprise (Cordis Neurovascular, Inc), the electrodetachable, fully retrievable Solo (eV3 Neurovascular) and the covered, balloon-expandable Willis (Microport). Development of significant focal stenosis remains a problem, seen in up to 5.8% of stent-assisted cases. A novel approach is to use a high-coverage, endoluminal mesh to divert flow and thus induce aneurysm thrombosis. The Pipeline Neuroendovascular Device (Chestnut Medical Technologies) is a tubular, bimetallic implant with approximately 30% coverage by area. Preliminary experience in humans has bees encouraging.

It is hoped that immunohistochemical and molecular biological data can be used to develop biologically active endovascular devices in the future. A novel method for depositing viable, migration capable fibroblasts on coils and successfully passing them through microcatheters may be a promising technique for endovascular intervention.

Intra-arterial (IA) therapies for acute stroke have evolved over the past decade. Despite the promising results of 1 PROACT 11, which demonstrated a 66% recanalization rate, substantially higher recanalization rates with IA pharmacologic thrombolysis have not been achieved over the past 7 years. The Food and Drug Administration recently approved a clot retrieval device (Merci retriever X5, X6; Concentric Medical, Mountain View, CA). Unfortunately, when used alone, the clot retriever is successful in only approximately 50% of cases, and multiple passes with this device are often required to achieve successful recanalization. IA thrombolytics administered concomitantly enhance the procedural success of this device but may increase the risk of hemorrhagic transformation of the reperfused infarction. There have been several reports of coronary stent implantation used for mechanical thrombolysis of recalcitrant occlusions. In a recent report, stent placement with balloon-mounted or self-expanding coronary stents was shown to be an independent predictor for recanalization of both intracranial and extracranial cerebrovascular occlusions. In another recent report, recanalization rates of 79% were achieved using balloon-mounted stent technology.

Self-expanding stents designed specifically for the cerebrovasculature can he delivered to target areas of intracranial stenosis with a success rate of >95% and an increased safety profile of deliverability because these stents are deployed at significantly lower pressures than balloon-mounted coronary stents. There has been an anecdotal report of the use of a self-expanding stent in the setting of acute symptomatic intracranial occlusion. In addition, a recent comparison of self-expanding and balloon-mounted stents in an animal model of acute embolic occlusion has shown no difference in the 2 stent groups with respect to recanalization rates.

This retrospective multicenter series demonstrates that the use of self-expanding stents is feasible in the setting of symptomatic medium- and large-vessel intracranial occlusions. With stent placement as a first-line mechanical treatment or as a "last-resort" maneuver, TIMI/TICI 2 or 3 revascularization was successfully obtained in 79% of the lesions in which stents were placed. This retrospective review suggests that focal occlusions limited to a single medium or large vessel, particularly solitary occlusions of the MCA for VBS, may be preferentially amenable to stent placement and thus can help clinicians to achieve improved rates of recanalization. In addition, gender may play a role in the success of self-expanding stent implantation: TIMI/TICI 2 or 3 flow was documented in all female patients studied, and female patients were more likely to achieve improved clinical outcomes as measured by NIHSS and mRS scores. Most importantly, our preliminary experience may lead to future pivotal studies that might aid clinicians to better stratify patients most likely to derive maximal clinical benefit from stent placement.

The use of other mechanical means has been reported to be effective in recanalization of acute occlusions. In the MERCI trial, overall recanalization rates TIMI/TICI 2 or 3 flow) of 48% were achieved with the Merci mechanical clot retriever.

Several authors have proposed endovascular treatment with stent deployment for ICA dissection with high-grade stenosis or occlusion when anticoagulation fails to prevent a new ischemic event. In these cases, the MCA was patent.

We found that stent-assisted endovascular thrombolysisithrombectomy compared favorably with IV rtPA thrombolysis.

This higher rate of MCA recanalization could be explained by carotid flow restoration, allowing direct access to the MCA thrombus.

Endovascular treatment could potentially extend the therapeutic window beyond 3 hours. Actually, in all cases in the endovascular group, MCA recanalization was obtained by 291 minutes.

Despite these promising preliminary results, potential drawbacks related to the procedure must be considered. Acute complications such as transient ischemic attack, ischemic stroke, femoral or carotid dissection, and death have been reported. Other potential hazards of endovascular treatment of carotid dissection could been observed, as they were in stenting of other cases of arteriopathy. In our series, 1 embolic stroke and 1 acute in-stent thrombosis occurred in the same patient. Despite this new infarction, we observed significant neurologic improvement in this patient, probably because the MCA remained patent. Late stent thrombosis has also been reported.

Document US 2007/0288054 A1 discloses the preamble of claim 1.

### SUMMARY OF THE INVENTION

Devices and systems facilitate and enable treatment of ischemic or hemorrhagic stroke. More specifically, a tethered basket-like system operates in conjunction with a microcatheter system, to provide arterial support and capture emboli.

According to a feature of the present invention, a device for the removal of emboli is disclosed in the appended claims, said device comprising a mesh capturer having at least an undeployed state and a deployed state, the mesh capturer being inserted into the neurovasculature in an undeployed state and removed from the microvasculature in its deployed state; wherein the mesh capturer is deployed into its deployed state distal to an embolus and advanced proximally until the embolus is substantially contained within the mesh capturer; and wherein the basket is deployed above the subclavian artery and common carotid artery.

According to a feature of the present disclosure, a method for removing an embolus is cited as an example not forming part of the invention, said method comprising inserting a microcatheter and guidewire distal to an embolus; inserting a embolus capture device over the wire through the microcatheter distal to the embolus; deploying the embolus capture device; retracting the deployed embolus capture device until the embolus is substantially contained within the embolus capture device; and removing the embolus capture device.

### BRIEF DESCRIPTION OF THE FIGURES

The above-mentioned features and objects of the present invention will become more apparent with reference to the following description taken in conjunction with the accompanying drawings wherein like reference numerals denote like elements and in which:
Fig. 1 shows a side view schematic of a microcatheter with wire passing an embolus;
Fig. 2 shows a cross-sectional schematic of an embodiment of a microcatheter wire passing by an embolus at a point of least resistance;
Fig. 3 is a side view of an embodiment of a device for capturing emboli according to the present invention comprising a basket for capturing the embolus;
Fig. 4 is a side view of an embodiment of a device for capturing emboli according to the present invention used as a safety device in a reperfusion operation; and
Fig. 5 is a flow diagram of an embodiment of a method wherein an embolus is removed from a patient after a reperfusion operation is unsuccessful.

### DETAILED DESCRIPTION OF THE INSTANT TEACHINGS

In the following detailed description of embodiments of the invention, reference is made to the accompanying drawings in which like references indicate similar elements, and in which is shown by way of illustration specific embodiments in which the invention may be practiced. These embodiments are described in sufficient detail to enable those skilled in the art to practice the invention, and it is to be understood that other embodiments may be utilized and that logical, mechanical, biological, electrical, functional, and other changes may be made without departing from the scope of the present invention. The following detailed description is, therefore, not to be taken in a limiting sense, and the scope of the present invention is defined only by the appended claims. As used in the present invention, the term "or" shall be understood to be defined as a logical disjunction and shall not indicate an exclusive disjunction unless expressly indicated as such or notated as "xor."

The ideal stent for intracranial use would he flexible, precisely delivered, retrievable, able to be repositioned, atraumatic, available in various lengths and diameters, thin-walled and radiopaque. It should provide sufficient coverage to restrain coils, while having wide enough fenestrations to permit catheterisation with coil or other embolic agent delivery catheters. The currently available over-the-wire stents are not ideal. The balloon-expandable stents of sufficient length are too stiff to be reliably and safely deployed. While existing self-expanding stents offer some improvement in this respect there are still serious difficulties in deploying them in distal locations and the currently available or planned stents for intracranial use are not available in the small diameters necessary for distal intracranial use.

The stent is delivered through a micro-catheter, allowing standard microcatheter/wire techniques to reach locations inaccessible to over-the-wire stents. It fulfils all the criteria mentioned above. A particularly appealing characteristic is its ability to be retrieved and repositioned after complete delivery, if its position is felt to be suboptimal or if the stent proves not to be necessary. The stent conforms completely to the normal vessel geometry and is not prone to strut opening on convexities. It is compatible with all currently used embolic agents for aneurysm occlusion and is MR compatible.

Stents have been used widely in occlusive lesions in the peripheral, renal, and coronary arteries to treat stenosis of vessels narrowed by a variety of pathologic conditions. Initially used mainly in extracranial cerebral vessels for carotid artery stenosis or the treatment of pseudoaneurysms of the extracranial carotid artery, small stents are now increasingly used for intracranial vessel disease such as the treatment of wide-necked aneurysms not amenable to conventional endovascular techniques.

Major limitations of the currently available stents, usually cardiac stents, however, are their relatively stiffness, rendering them not flexible enough to pass the Cl /C2 vertebral artery or carotid siphon tortuousities.

The design constraints for the device used in this study were to develop an endovascular stent that is flexible enough to be delivered via a microcatheterand to be placed in small vessels but with sufficient radial forces to conform to the vessel wall when deployed.

Leaving the guidewire in place after stent deployment in curved vessels might be an option to stabilize the stent and thus prevent stent displacement while catheterizing the aneurysm with a second microcatheter. In contrast to the Neuroform, because of the design of our stent displacement should not be an issue.

The present inventors have realized that by leveraging a conventional self-expanding reperfusion device delivery platform, a poly-modic system can be iterated which crosses a embolus, filters, and either removes the offending embolus or is optionally emplaced to address the same. A paucity of extant systems effective for such combination therapies is noted among the art. The instant system allows for natural lysis, perfusion of the challenged vessels, and importantly filters any particulates generated, to obviate the need to be concerned with distal migration of the particulates generated.

The present invention relates to emboli removal devices used to treat, among other things, ischemic stroke. Naturally, therefore, the emboli removal devices of the present invention are designed to be used in neuro-type applications, wherein the specifications of the present catheters and emboli removal devices may be deployed in the blood vessels of the cerebral vascular system. Similarly contemplated for the emboli removal systems and catheters of the present invention is deployment in other parts of the body wherein the specifications of the present invention may be used in other vessels of the body in a non-invasive manner, in examples not forming part of the invention as claimed. Specifically, the inventors of the present invention have devised devices and methods of the removal of neurocranial emboli without causing distal complication arising from the passing of larger pieces of a recovered embolus distal to the location of the original embolus.

According to embodiments, disclosed herein is a catheter-emboli removal system. The emboli removal devices of the present invention are for reperfusion of blood vessels. When the catheter-emboli removal system of the present invention is deployed into a blood vessel having an embolus, the emboli removal device is expanded and moved proximally along the vessel so that the embolus is substantially contained with the mesh basket of the emboli removal device.

According to the instant teachings, deployment of the system of the present invention establishes immediate 50% of the diameter of the lumen patency of the vessel being addressed by removing the embolus occluding the vessel. Among the prior art, no system having adequately small profile with flexibility to promote improved access for in-site treatment is known which may be used as a temporary (not implanted) solution and removed without substantial damage to the vasculature.

Additionally, in reperfusion applications the emboli removal device may be deployed as a safety device. As the embolus lyses, the deployed emboli removal device filters larger embolus particles from migrating distally, thereby reducing the chances of further complications. If reperfusion is unsuccessful, then the emboli removal device is retracted proximally, thereby substantially capturing the embolus. Then the entire device is removed together with the microcatheter.

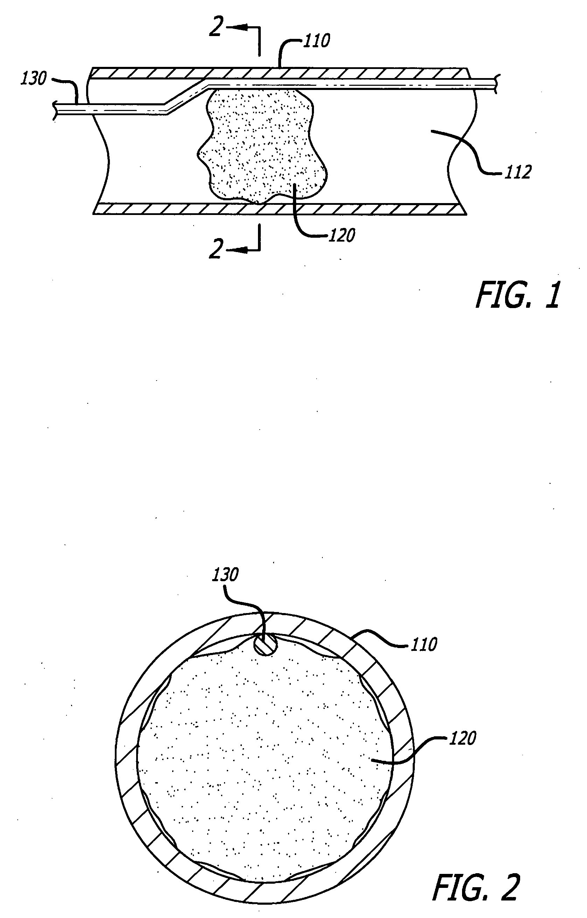

According to embodiments and as illustrated in Fig. 1, a cross sectional view of an artery 110 having embolus 120 in artery lumen 112 is shown. Guidewire 130 inserted through a thrombus tends to follow the path of least resistance through the softest parts of embolus 120. When a microcatheter is inserted along guidewire 130, it likewise follows this path of least resistance. Accordingly, when a stent or embolus capture device is inserted via guidewire 130, it is deployed offset because guidewire 130 is not centered in the vessel in many cases, as illustrated in Fig. 2. Those skilled understand how struts of the line subject devices enable recapturability, flexibility and tracking.

To address the problem of the guidewire offset, the inventors devised an embolus capture device or basket 200 that is adept at capturing embolus 120 even when deployed in an offset way. As part of the embolus capture device/basket 200 design, pieces of embolus 120 that break away from embolus 120 are recaptured to prevent potential migration more distal in the vasculature which may potentially cause other emboli, too remote to safely address.

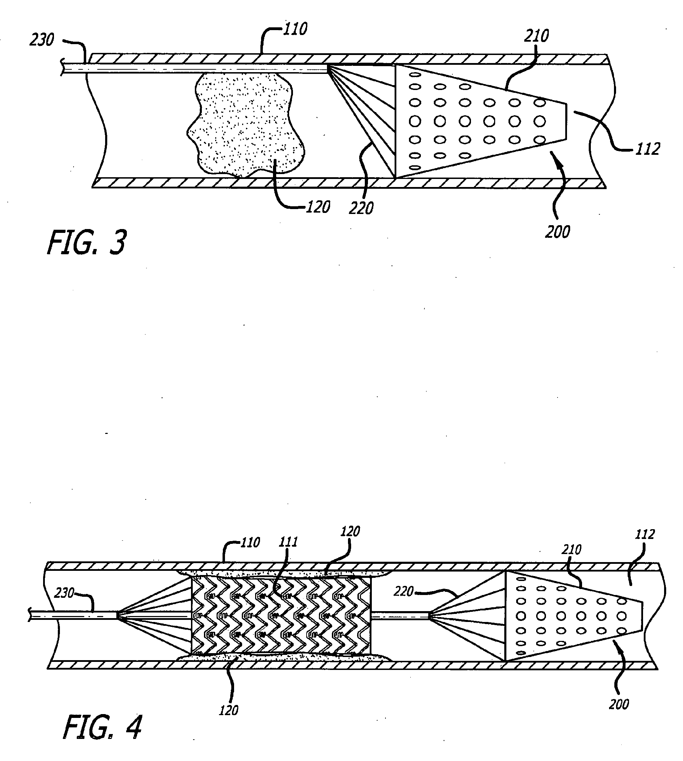

As illustrated in Fig. 3, blood vessel 110 is shown having vessel lumen 112 and embolus 120. As illustrated, embolus capture device/basket 200 is deployed for capture of embolus 120. As illustrated, embolus capture device/basket 200 is deployed along an offset guidewire. However, embolus capture device 200 is designed for offset deployment to deploy such that it occupies about the center of vessel 110, which ensure maximum efficiency in the capture of embolus 120. It will be readily recognized that the devices of the present invention need not be deployed offset.

Embolus capture device/basket 200 comprises mesh basket 210 and tethers 220 which are deployed from microcatheter 230. Mesh basket 210 comprises a radially expandable woven mesh or coil basket open on the proximal end and closed at the distal end. The mesh may be made from materials well known and understood by artisans, including polymers, flouropolymers, nitinol, stainless steel, vectran, or kevlar. Other biocompatible materials that may be woven or coiled are similarly contemplated. Mesh basket 210 connects to microcatheter 230 via tethers 220 and is designed to be compatible such that it is removable in its deployed state without causing dissection or other damage to the vasculature.

Mesh basket 210 comprises a plurality of individual cells, having a uniform size or spacing geometry or a variable size or spacing geometry. According to embodiments where the size or spacing geometry is variable, smaller size or spacing geometry is used to provide a tight mesh for preventing the passage of small pieces of embolus 120 that break away. Larger size or spacing geometry units allow from blood flow 110. In all cases, size or spacing geometry will not allow pieces of embolus 120 that may cause potential complications.

Tethers 220 serve to provide structure for mesh basket 110, while providing large openings whereby blood may freely flow from the proximal to distal end of embolus removal device/basket 200. According to embodiments, tethers 220 are made from the same material as mesh basket 210. Those skilled in the art will readily understand that materials for tethers and mesh may be the same, different, or interchangeable, as needed.

During deployment of embolus capture device/basket 200, mesh basket is stored in microcatheter 230 in an undeployed state. In the undeployed state, microcatheter 230 is advanced distal to embolus 120 and mesh basket 210 is deployed. According to embodiments, both mesh basket 210 and tethers 220 are deployed distal to embolus 120 to prevent tethers 220 from dislodging pieces of embolus 120 prior to full expansion of mesh basket 210, thereby preventing the pieces from advancing distal to the embolus 120 before mesh basket 210 is in place to filter them.

After deployment, according to embodiments, embolus removal system 200 is retracted proximally until embolus is substantially contained within mesh basket 210. Thereafter, mesh basket 210 and microcatheter 230 are removed from the vasculature of the patient. During removal of mesh basket 210 and microcatheter 230, embolus 120 is trapped within mesh basket 210 and withdrawn from vessel 110.

According to embodiments, microcatheter 230 length and diameter are suitable for inserting into a human patient and capable of reaching a target embolus in the region above the subclavian and common carotid arteries. For example, according to embodiments, microcatheter 230 is about 150 cm long; microcatheter has a proximal segment (at a control end of microcatheter 230) that is about 115 cm long with an outer diameter of about 3.5 French and a distal segment (at a deployment end of microcatheter 230) is about 35 cm with an outer diameter of about 2.7 French. The inventors contemplate, according to embodiments a gradual decrease or stepwise in the outer diameter dimension as a function of the distal distance from proximal segment, according to embodiments. For example, proximal segment is 3.5 French at the most proximal end and distal segment is 2.7 French at the most distal end. Disposed between is a segment having one or more intermediate outer diameters between 3.5 French and 2.7 French, such as 3.2 French and 3.0 French. The inner diameter of microcatheters 230 is 0,30 to ,0,74 mm (0.012 to .029 inches), according to embodiments, which allows microcatheter to be inserted along a preinserted guidewire or used to infuse therapeutic agents. According to embodiments, the performance of microcatheter 230 is comparable to standard microcatheters 230 and is designed to track over a guidewire through the neuro-vasculature.

As illustrated by embodiments in Fig. 4, embolus capture device/basket 200 may be deployed concurrently with a tethered reperfusion device 111. As embolus 120 is reperfused with reperfusion device 111, embolus capture device/basket 200 provides a safety feature whereby pieces of embolus 120 that break away are captured in mesh basket 210 and removed with the reperfusion device generally. Additionally, as vessel 110 reperfuses due to natural lytic action, mesh basket 210 provides a minimum particle size permitted to pass distal to embolus capture device/basket 200. Consequently, embolus capture device/basket 200 prevents further complications distal to the original site of the occlusion by preventing larger embolus 120 pieces or particles from passing deeper into the neurovasculature and occluding it in more distal locations.

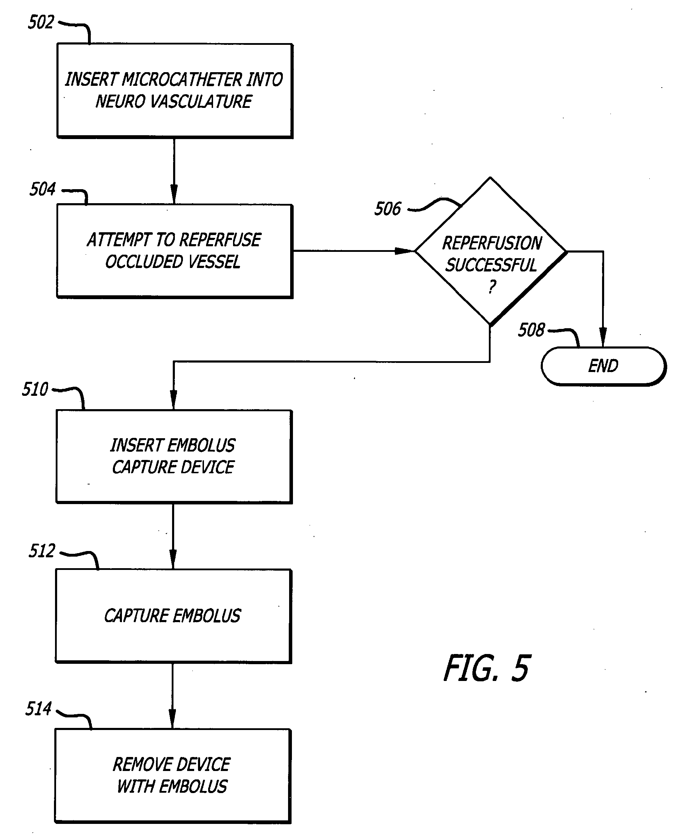

Alternately and as illustrated according to embodiments in Fig. 5, reperfusion device is used after reperfusion is unsuccessfully attempted or not successful to the desired level. Accordingly, microcatheter is inserted into the neurovasculature in operation 502 as well known and understood by artisans. Reperfusion is attempted, for example with the reperfusion device 210 of Fig. 4 in operation 504 of Fig. 5. After reperfusion is attempted, the success is determined in operation 506. For example, a contrast dye is used to determine the level to which the occluded vessel is reperfused, as is well known to artisans.

If reperfusion is not successful to a desired degree, then embolus capture device/basket 200 is inserted through the microcatheter as described herein and deployed distal to the embolus 120. For example, creating a channel for flow ideally includes making a vessel at least about halfway-patant, or 50% of diameter of a vessel being open. According to embodiments, the channel created may be a cerebral equivalent of thrombolysis in myocardial infarction (TIMI) 0, TIMI 1, or TIMI 2, TIMI 3, and thrombolysis in cerebral infarction (TICI) and TICI 3In these cases, blood flow is not accomplished to a desired degree. It is therefore desirable to remove the entire embolus. Thus, after embolus capture device/basket 200 is deployed distal to the embolus, it is retreated proximal until embolus 120 is substantially inside of mesh basket 210 in operation 512. Thereafter, mesh basket 210, embolus 120, and microcatheter 230 are removed.

The embolus capture devices of the present invention may be designed for over the wire deployment or rapid exchange deployment, according to embodiments.

U.S. Letters Patents Number 5,928,260 and 5,972,219 along with 7,147,655; 7,160,317; 7,172,575; 7,175,607; and 7,201,770 are cited herein as background art.

While the apparatus and method have been described in terms of what are presently considered to be the most practical and preferred embodiments, it is to be understood that the invention need not be limited to the disclosed embodiments. It is intended to cover various modifications and similar arrangements included within the scope of the claims, the scope of which should be accorded the broadest interpretation so as to encompass all such modifications and similar structures. The present invention includes any and all embodiments of the following claims.

It should also be understood that a variety of changes may be made without departing from the scope of the appended claims. It should be understood that this invention is intended to yield a patent covering numerous aspects of the invention both independently and as an overall system and in apparatus mode.

Further, each of the various elements of the invention and claims may also be achieved in a variety of manners. This invention should be understood to encompass each such variation, be it a variation of an embodiment of any apparatus embodiment, a process embodiment, or even merely a variation of any element of these.

In this regard it should be understood that for practical reasons and so as to avoid adding potentially hundreds of claims, the applicant has presented claims with initial dependencies only.

## Claims

1. A reperfusion system comprising:
a microcatheter (230) configured to be inserted into the neurovasculature;
an embolus capture device (200) having an undeployed state and a deployed state and being stored in the microcatheter (230) in the undeployed state, wherein the embolus capture device (200) comprises a mesh basket (210) and tethers (220) which are deployed from the microcatheter (230); and
wherein, in use, the microcatheter (230) can be inserted into the neurovasculature and can be advanced distal to an embolus and the embolus capture device (200) can be deployed into its deployed state; and **characterized in that** said system further comprises a self-expanding tethered reperfusion device (111) for reperfusion of an embolus;
wherein, in use, the tethered reperfusion device (111) can be deployed concurrently with the embolus capture device (200), such that the embolus capture device (200) can provide a safety feature whereby pieces of embolus that break away are captured and removed with the reperfusion device (111).

2. The system of claim 1, wherein, in use, the embolus capture device (200) is deployed above the subclavian artery and common carotid artery.

3. The system of claim 1 or claim 2, wherein the embolus capture device (200) has an eccentric configuration to improve capture of the embolus.

4. The system of any previous claim, wherein the mesh basket (210) comprises a radially expandable woven mesh or coil basket having an open proximal end and a closed distal end to enable capture of the embolus.

5. The system of any previous claim, wherein the mesh basket (210) comprises a plurality of individual cells having a uniform size or spacing geometry.

6. The system of any of claims 1 to 4, wherein the mesh basket (210) comprises a plurality of individual cells having a variable size or spacing geometry.

7. The system of claim 6, wherein smaller size or spacing geometry is used to provide a tight mesh for preventing the passage of small pieces of the embolus that break away.

8. The system of any previous claim, wherein the embolus capture device (200) is configured to be deployed over a guidewire (130).

9. The system of any of claims 1 to 7, wherein the embolus capture device (200) is configured to be deployed as a component of a rapid exchange system.

10. The system of any previous claims, wherein the mesh basket (210) is connected to the microcatheter via tethers (220).

11. The system of any previous claim, wherein the mesh basket (210) is made from a material selected from the group consisting of: polymers; fluoropolymers; Nitinol; stainless steel; vectran; and Kevlar.

## Patentansprüche

1. Reperfusionssystem, umfassend:
einen Mikrokatheter (230), der derart konfiguriert ist, um in das neurovaskuläre System eingeführt zu werden;
eine Embolusfangvorrichtung (200), die einen nicht entfalteten Zustand und einen entfalteten Zustand aufweist und in dem nicht entfalteten Zustand in dem Mikrokatheter (230) gelagert wird, wobei die Embolusfangvorrichtung (200) einen Gitterkorb (210) und Halteschnüre (220) umfasst, die sich aus dem Mikrokatheter (230) entfalten; und
wobei der Mikrokatheter (230) bei der Verwendung in das neurovaskuläre System eingeführt werden kann und distal zu einem Embolus vorangetrieben werden kann, und die Embolusfangvorrichtung (200) in ihren entfalteten Zustand entfaltet werden kann; und**dadurch gekennzeichnet, dass** das System ferner eine sich selbst ausbreitende, mit Halteschnüren versehene Reperfusionsvorrichtung (111) zur Reperfusion eines Embolus umfasst;
wobei die mit Halteschnüren versehene Reperfusionsvorrichtung (111) bei der Verwendung gleichzeitig mit der Embolusfangvorrichtung (200) entfaltet werden kann, sodass die Embolusfangvorrichtung (200) ein Sicherheitsmerkmal bereitstellen kann, wodurch abbrechende Teile des Embolus eingefangen werden und mit der Reperfusionsvorrichtung (111) entfernt werden.

2. System nach Anspruch 1, wobei die Embolusfangvorrichtung (200) oberhalb der Arteria subclavia und der Arteria carotis communis entfaltet wird.

3. System nach Anspruch 1 oder 2, wobei die Embolusfangvorrichtung (200) eine exzentrische Konfiguration aufweist, um das Fangen des Embolus zu verbessern.

4. System nach einem der vorhergehenden Ansprüche, wobei der Gitterkorb (210) einen sich radial ausbreitenden Gitternetz- oder Rohrschlangenkorb umfasst, der ein offenes proximales Ende und ein geschlossenes distales Ende aufweist, um das Fangen des Embolus zu ermöglichen.

5. System nach einem der vorhergehenden Ansprüche, wobei der Gitterkorb (210) eine Vielzahl an einzelnen Zellen umfasst, die eine einheitliche Größe oder Abstandsgeometrie aufweisen.

6. System nach einem der Ansprüche 1 bis 4, wobei der Gitterkorb (210) eine Vielzahl an Zellen umfasst, die eine variable Größe oder Abstandsgeometrie aufweisen.

7. System nach Anspruch 6, wobei eine kleinere Größe oder Abstandsgeometrie verwendet wird, um ein engmaschiges Gitter zur Verhinderung des Durchtritts kleiner, von dem Embolus abbrechender Teile bereitzustellen.

8. System nach einem der vorhergehenden Ansprüche, wobei die Embolusfangvorrichtung (200) derart konfiguriert ist, um über einem Führungsdraht (130) entfaltet zu werden.

9. System nach einem der Ansprüche 1 bis 7, wobei die Embolusfangvorrichtung (200) derart konfiguriert ist, um als Komponente eines Schnellwechselsystems entfaltet zu werden.

10. System nach einem der vorhergehenden Ansprüche, wobei der Gitterkorb (210) über Halteschnüre (220) mit dem Mikrokatheter verbunden ist.

11. System nach einem der vorhergehenden Ansprüche, wobei der Gitterkorb (210) aus einem Material besteht, das aus der Gruppe, die aus Polymeren, Fluorpolymeren, Nitinol, Edelstahl, Vectran und Kevlar besteht, ausgewählt ist.

## Revendications

1. Système de reperfusion comprenant :
un micro-cathéter (230) configuré pour être inséré dans le système neurovasculaire ;
un dispositif de capture (200) d'embole ayant un état non déployé et un état déployé et étant stocké dans le micro-cathéter (230) à l'état non déployé, dans lequel le dispositif de capture (200) d'embole comprend un panier à mailles (210) et des lignes d'attache (220) qui sont déployées depuis le micro-cathéter (230) ; et
dans lequel, lors de l'utilisation, le micro-cathéter (230) peut être inséré dans le système neurovasculaire et peut être avancé de manière distale à un embole et le dispositif de capture (200) d'embole peut être déployé dans son état déployé ; et **caractérisé en ce que** ledit système comprend en outre un dispositif de reperfusion (111) attaché auto-extensible pour la reperfusion d'un embole ;
dans lequel, lors de l'utilisation, le dispositif de reperfusion (111) attaché peut être déployé en même temps qu'avec le dispositif de capture (200) d'embole, de sorte que le dispositif de capture (200) d'embole puisse fournir une caractéristique de sécurité, moyennant quoi des morceaux d'embole qui se détachent sont capturés et retirés à l'aide du dispositif de reperfusion (111).

2. Système selon la revendication 1, dans lequel, lors de l'utilisation, le dispositif de capture (200) d'embole est déployé au-dessus de l'artère subclavière et de l'artère carotide commune.

3. Système selon la revendication 1 ou la revendication 2, dans lequel le dispositif de capture (200) d'embole présente une configuration excentrique pour améliorer la capture de l'embole.

4. Système selon une quelconque revendication précédente, dans lequel le panier à mailles (210) comprend un panier hélicoïdal ou à mailles tissé radialement extensible ayant une extrémité proximale ouverte et une extrémité distale fermée pour permettre la capture de l'embole.

5. Système selon une quelconque revendication précédente, dans lequel le panier à mailles (210) comprend une pluralité de cellules individuelles ayant une géométrie d'espacement ou de taille uniforme.

6. Système selon l'une quelconque des revendications 1 à 4, dans lequel le panier à mailles (210) comprend une pluralité de cellules individuelles ayant une géométrie d'espacement ou de taille variable.

7. Système selon la revendication 6, dans lequel une géométrie d'espacement ou de taille plus petite est utilisée pour fournir une maille étroite pour empêcher le passage de petits morceaux de l'embole qui se détachent.

8. Système selon une quelconque revendication précédente, dans lequel le dispositif de capture (200) d'embole est configuré pour être déployé par-dessus un fil-guide (130).

9. Système selon l'une quelconque des revendications 1 à 7, dans lequel le dispositif de capture (200) d'embole est configuré pour être déployé comme composant d'un système d'échange rapide.

10. Système selon une quelconque revendication précédente, dans lequel le panier à mailles (210) est connecté au micro-cathéter par le biais des lignes d'attache (220).

11. Système selon une quelconque revendication précédente, dans lequel le panier à mailles (210) est composé d'un matériau sélectionné dans le groupe constitué de : polymères ; fluoropolymères ; Nitinol ; acier inoxydable ; vectran ; et Kevlar.
